(19) Europäisches Patentamt
European Patent Office
Office européen des brevets

(11) Numéro de publication: **0 191 659 B1**

(12) **FASCICULE DE BREVET EUROPEEN**

(45) Date de publication du fascicule du brevet: **11.04.90**

(51) Int. Cl.[5]: **C 07 H 19/20**, C 12 Q 1/68, C 07 H 21/00

(21) Numéro de dépôt: **86400032.8**

(22) Date de dépôt: **08.01.86**

(54) **Nouveaux nucléosides de dérivés de l' adénosine, leur préparation et leurs applications biologiques.**

(30) Priorité: **08.01.85 FR 8500203**

(43) Date de publication de la demande:
**20.08.86 Bulletin 86/34**

(45) Mention de la délivrance du brevet:
**11.04.90 Bulletin 90/15**

(84) Etats contractants désignés:
**AT BE CH DE FR GB IT LI LU NL SE**

(56) Documents cités:
**EP-A-0 176 396**

**TETRAHEDRON LETTERS, vol. 25, no. 19, 1984, pages 1975-1978, Pergamon Press Ltd., GB; A.H. BEITER et al.: "Solution synthesis of protected DI-2'-deoxynucleoside phosphotriesters via the phosphoramidite approach"**

**CHEMICAL ABSTRACTS, vol. 80, no. 17, 29 avril 1974, page 446, résumé no. 96289b, Columbus, Ohio, US; Z. KAZIMIERCZUK et al.: "Preparative photochemical synthesis of isoguanosine ribo- and deoxyribonucleosides and nucleotides, and isoguanosine cyclic 3'5'-phosphate a new cyclic AMP analog" & ACTA BIOCHIM. POL. 1973,20(4), 395-402; & ABSTRACTS ET CHEMICAL**

(73) Titulaire: **INSTITUT PASTEUR**
**25-28, rue du Docteur Roux**
**F-75724 Paris Cédex 15 (FR)**

(73) Titulaire: **CENTRE NATIONAL DE LA RECHERCHE SCIENTIFIQUE (CNRS)**
**15, Quai Anatole France**
**F-75007 Paris (FR)**

(72) Inventeur: **Huynh Dinh, Tam**
**32, rue Paul Déroulède**
**F-78290 Croissy/Seine (FR)**
Inventeur: **Gouyette, Catherine**
**19, avenue de Verdun**
**F-92170 Vanves (FR)**
Inventeur: **Igolen, Jean**
**4, rue Croix Mathurine**
**F-78320 le Mesnil St-Denis (FR)**

(74) Mandataire: **Peaucelle, Chantal**
**S.C. Ernest Gutmann - Yves Plasseraud 67, boulevard Haussmann**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 191 659 B1**

(56) Documents cités:
**ABSTRACTS NINTH COLLECTIVE INDEX, page 33171CS: 2H-Purin-2-one, -,6-amino-1,9-dihydro-9-(2-O-phosphono-beta-D-ribofuranosyl) - ammonium salt, 80: 96289b and -,6-amino-1,9-dihydro-9-(5-O-phosphono-beta-D-ribofuranosyl)-, 80: 96289b**

**Nucleic Acids Research, Vol. 16 (1988), pages 305-317**

## Description

L'invention a pour objet de nouveaux nucléosides de dérivés de l'adénosine, leur préparation et leurs applications biologiques, plus spécialement pour l'élaboration de séquences de nucléotides utilisables en particulier comme sondes dans le domaine des analyses et extractions biologiques.

Dans la demande FR 84 13095 du 22.08.1984 au nom des demandeurs, on a décrit des sondes constituées de fragments d'oligonucléotides comportant, en tant que bases puriques, des groupes adénine modifiés, à savoir des groupes adénine capables de former trois liaisons hydrogène avec les groupes réactifs des bases pyrimidiques (thymine, uracile).

Les groupes adénine en question sont avantageusement modifiés par introduction sur le carbone en position 2 (sommet C—2) du cycle pyrimidine d'un groupement tel que $—NH_2$, —OH ou —SH, le groupe $—NH_2$ étant particulièrement préféré.

Selon un mode de réalisation préféré des sondes ci-dessus, une partie au moins des groupes adénine des fragments d'oligonucléotides répond à la formule (a):

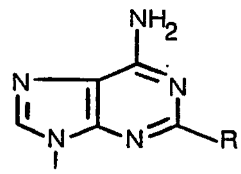

(a)

dans laquelle *R* représente un groupe $—NH_2$, —OH ou —SH.

Les fragments d'oligonucléotides constituant ces sondes sont préparés selon les méthodes classiques, avantageusement en phase solide, par couplage d'un groupe en position 3' d'un motif sucre d'un nucléotide donné avec un groupe en position 5' du motif sucre d'un aute nucléotide, éventuellement fixé à un support polymère.

Par expression "motif sucre", on désigne dans la description et les revendications aussi bien un motif ribose qu'un motif desoxyribose.

Dans Tetrahedron Letters, vol. 25 n° 19, p. 1975—1978, 1984, on décrit la synthèse de di-2'-deoxyribonucléoside-3'-2-(4-nitrophenyl)ethyl phosphates selon la voie phosporamidite en solution.

Les développements des travaux des inventeurs dans ce domaine pour disposer de nucléosides d'obtention aisée et dotés d'une réactivité élévée les ont amenés à constater qu'en introduisant des dérivés de phosphore déterminés sur les motifs sucre des groupements adénine modifiés ci-dessus, il est possible de disposer d'une famille de nucléosides dotés de propriétés avantageuses notamment d'une grande réactivité, ce qui les rend particulièrement intéressants pour la synthèse de séquences nucléotidiques longues.

L'invention a donc pour but de fournir de nouveaux dérivés de nucléosides de bases puriques permettant de mettre à profit les avantages des adénines modifiées déjà mises au point tout en disposant de produits possédant des propriétés améliorées, notamment, une plus grande réactivité.

Elle vise également à fournir un procédé permettant d'obtenir ces dérivés en une étape.

L'invention a également pour but l'application de ces dérivés pour l'élaboration avec des rendements élevés de séquences d'oligonucléotides utilisables en biologie notamment comme sondes de détection.

Les dérivés de l'invention qui comportent, comme défini ci-dessus, des groupements adénine capables de former, après déblocage des fonctions réactives, trois liaisons hydrogène avec les groupes réactifs des bases pyrimidiques (thymine, uracile), sont caractérisés en ce que le motif sucre, est substitué en position 3' ou en position 5' par un groupe phosphite ou phosphoramidite de formule I,

$$—O—\underset{\displaystyle OR_1}{\underset{|}{P}}—X \qquad (I)$$

dans laquelle:

$R_1$ représente un radical alcoyle inférieur, en particulier un radical méthyle, ou $—(CH_2)_n—CN$, *n* étant un nombre de 1 à 4;

*X* représente un atome d'halogène ou un groupe amine primaire, secondaire ou tertiaire.

Dans un groupe préféré, *X* représente un atome d'halogène en particulier de chore ou de brome.

Dans un autre groupe préféré, *X* représente un groupe amine, plus specialement un groupe amine tertiaire. Des groupes particuliers appropriés pour la mise en oeuvre de l'invention comprennent les radicaux diméthylamino, diéthylamino, dipropylamino, diisopropylamino, morpholino, pyrrolidino, et 2,2,6,6-tétraméthylpipéridino.

Dans un autre groupe préféré, *X* représente un groupe amine secondaire résultant de l'élimination de l'atome d'hydrogène du cycle azoté d'un hétérocycle azoté contenant des liaisons insaturées dans le cycle tels que le tétrazole, l'imidazole, des imidazoles substitués du type du nitroimidazole, l'indole, le pyrazole, le benzimidazole, l'isoindole, le pyrrole, le triazole, le dioxazole et analogues.

D'une manière avantageuse, la substitution des motifs sucre liés aux adénines modifiées par le groupe phosphoramidite de formule I confère aux produits correspondants une grande réactivité ce qui permet d'élaborer aisément des séquences longues de nucléodides et de les utiliser sur les machines classiques de synthèse d'oligo et polynucléotides.

Les analyses par RMN mettent, en outre, en évidence la pureté élevée de ces produits ce qui les rend précieux pour obtenir des rendements de couplage élevés dans la synthèse des oligonucléotides.

De préférence, les groupes adénine des dérivés de l'invention sont modifiés par introduction sur le sommet C—2 du cycle pyrimidine d'un groupement susceptible, après déblocage de donner une liaison hydrogène avec l'atome d'oxygène fixé sur le sommet C—2 de la thymine ou de l'uracile.

Selon un mode préféré de réalisation de l'invention, les dérivés de nucléosides comprennent sur le sommet C—2 du cycle pyrimidine des groupements adénine, a) un groupe amino, b) un groupe hydroxyle —OH, ou *c*), un groupe sulfhydrile —SH ces groupes étant bloqués par remplacement d'un atome d'hydrogène, ou des deux atomes d'hydrogène dans le cas du groupe amino, par un groupe protecteur tel qu'utilisé habituellement pour ces types de fonction.

Dans une famille préférée de nucléosides, les dérivés comportent un groupe amino sur le sommet C—2 et répondent à la formule I:

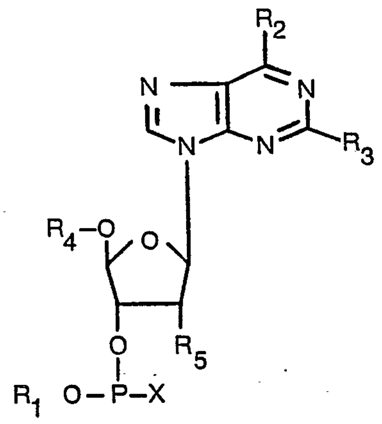

(II)

dans laquelle:

$R_1$ et $X$ sont tels que définis ci-dessus, $X$ présentant avantageusement les significations préférées des différents groupes considérés,

$R_2$ représente un groupe $—NH(A_1)$ ou $—N(A_1, A_2)$,

$R_3$ présente les significations de $R_2$ ou représente un groupe $—O(A_1)$ ou $—S(A_1)$,

$A_1$ et $A_2$ identiques ou différents l'un de l'autre étant choisis parmi les groupes acyle, notamment acétyle et benzoyle, butyryle, isobutyryle et autre groupe analogue pouvant être éliminé sans altération des motifs nucléosidiques ou de la chaîne nucléotidique formée à partir de ces motifs,

$R_4$ représente un groupe de blocage de la fonction —OH en position 5', en particulier, un groupe choisi parmi les radicaux trityle, méthoxytrityle, diméthoxytrityle, dialcoxyphosphite, pivalylisobutyloxycarbonyle t-butyldiméthylsilyle, et

$R_5$ représente un atome d'hydrogène ou un groupe $—OR_6$ avec $R_6$ présentant les significations de $A_1$, $A_2$ ou $R_4$.

Dans un groupe préféré de cette famille, $R_3$ représente un groupement amino protégé par $A_1$ et, le cas échéant, $A_2$.

Dans un autre groupe préféré, $R_3$ représente un groupement —OH protégé, le cas échéant comme indiqué ci-dessus.

Un autre groupe préféré encore comprend les dérivés dans lesquels $R_3$ représente un groupement $—SA_1$.

Conformément à l'invention, les dérivés de nucléosides définis ci-dessus sont obtenus par réaction, à l'abri de l'air, a) d'un dérivé d'adénosine de formule (III) substitué par les groupements réactifs souhaités, ces groupements étant protégés et comportant un groupe —OH libre en position 3' du motif sucre, avec b) un phosphite de formule (IV). Cette réaction qui conduit aux nucléosides protégés de formule (V) peut être schématisée comme suit:

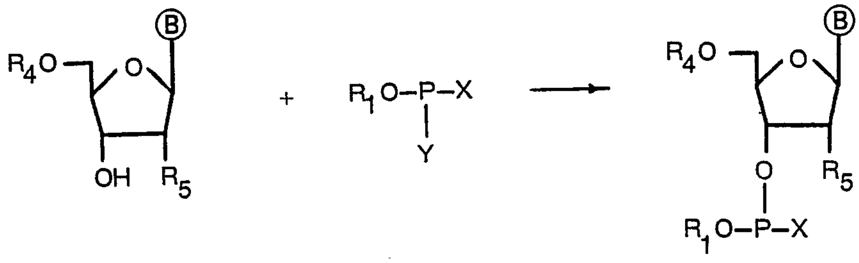

(III) (IV) (V)

Dans ces formules *X* et $R_1$ sont tels que définis ci-dessus, *y* représente un groupe réactif capable de réagir avec le groupe —OH libre du motif sucre, établissant une liaison entre O et P, et en formant un composé YH facilement éliminable et *B* représente une adénine modifiée.

Le principe de la réaction ci-dessus s'applique à la synthèse des nucléosides renfermant un groupe phosphoramidite en position 5', mais en utilisant un nucléoside de formule III ci-dessus dont le groupe —OH en 5' est libre et le groupe —OH en 3' est bloqué, notamment par un substituant du type de $R_4$.

Dans des dérives avantageux, compte-tenu de leur réactivité élevée et de leur facilité d'obtention, *Y* représente un atome d'halogène plus spécialement un atome de chlore.

De préference *X* représente un groupement —N,N-diisopropyle: —N $[CH(CH_3)_2]$, —N— morpholino:

- 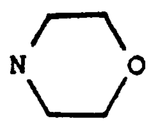,-N-pyrrolidino : -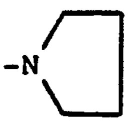 ou 2,2,6,6-tétraméthyl-N-pipéridino : -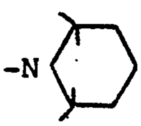

et $R_1$ représente un radical alcoyle, plus spécialement un radical méthyle ou $-(CH_2)_n-CN$, *n* étant un entier de 1 à 4, de préférence égal à 2.

Pour préparer les dérivés dans lesquels la base *B* comporte un groupe $-NH_2$ sur le sommet C—2, il est plus spécialement avantageux d'utiliser la O-diméthoxytrityl-5' N,N'-diisobutyryl amino-2 désoxy-2' adénosine préparée à partir de la désoxy-2' guanosine, par analogie avec la technique décrite par W. L. Sung dans J. Chem. Soc. (Communication, 1089 (1981) en relation avec la thymidine.

La préparation des dérivés dans lesquels la base *B* est modifiée sur le sommet C—2 de la pyrimidine par un groupe —OH est réalisée de manière satisfaisante par la O-diméthoxytrityl-5' N-isobutyryl hydroxy-2 désoxy-2' adénosine qui peut être préparée également par analogie avec la méthode de J. Davoll dans J.A.C.S. 1951, 73, p. 3174 et dans "in nucleic acid Chemistrv" L. D. Townsend, R. S. Tipson in part 2 p. 565, John Wiley, N.Y. 1978.

Dans le cas des dérivés renfermant une base *B* modifiée sur le sommet C—2 en question par un groupe —SH, on utilise avantageusement la O-diméthoxytrityl-5' N-isobutyryl mercapto-2 désoxy-2' adénosine qui peut être préparée de la même manière que le dérivé hydroxy-2 correspondant, en remplaçant, de manière connue en soi, le groupe OH par un groupe SH.

Les phosphochloridites préférés en raison de leur volatilité réduite et par là de leur plus faible réactivité avec l'humidité atmosphérique comprennent les dérivés suivants:

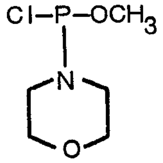 et 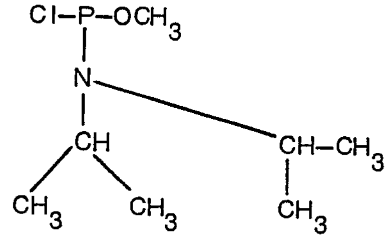

Selon une disposition avantageuse de 'invention, on utilise un excès de phosphite: cet excès est de préférence supérieur à environ 2, et plus spécialement de l'ordre 3.

La réaction entre les composés III et IV est effectuée de préférence en présence d'une base, en particulier, d'une amine organique.

Selon une autre disposition la réaction est effectuée à température ambiante, jusqu'à disparition du dérivé d'adénosine de départ, ce que est aisément vérifiable par exemple par chromatographie sur couche mince.

Pour faciliter a réaction, le dérivé d'adénosine protégé est mis en solution dans un solvant organique tel que le dichlorométhane, le tétrahydrofuranne, l'acétonile ou le dioxane.

Le produit obtenu est avantageusement purifié.

Le processus de purification peut comprendre une ou plusieurs des étapes de lavage, de séchage, filtration, évaporation à sec suivie d'une reprise dans un solvant organique du type du toluène, précipitation à l'aide d'un solvant organique du type de l'éther de pétrole, de centrifugation, séchage du précipité et chromatographie sur colonne du précipité récupéré lors de la centrifugation.

Les nucléosides obtenus constituent des intermédiaires de synthèse particulièrement intéressants en raison notamment de leur réactivité élevée. Ils permettent d'obtenir des rendements de couplage supérieurs à 95% lors de la constrution de séquences de nucléotides et sont utilisables aussi bien pour la synthèse manuelle que pour la synthèse automatisée de ces séquences.

Avantageusement, ces séquences d'oligonucléotides renfermant ou constituées par ces nucléosides sont capables de s'hybrider avec une grande stabilité et une sensibilité élevée avec une séquence de nucléotides complémentaires et constituent donc des sondes de grand intérêt pour détecter et analyser des séquences de nucléotides d'une composition donnée, notamment dans le domaine des diagnostics microbiologiques, des défauts génétiques ou de tout autre domaine mettant en oeuvre une hybridation entre acides nucléiques.

Elles sont particulièrement appropriées pour remplacer les sondes mixtes pour la détection et l'isolement de séquences de nucléotides, notamment, d'ARN-messagers (ARN-m) ou d'ADN-complémentaires (c-ADN).

De façon particulièrement avantageuse cette sonde donne, en effet, une hybridation analogue à celle d'une sonde mixte et confère une sensibilité analogue dans la détection après hybridation avec des séquences complémentaires en réalisant une séquence déduite d'une séquence particulière d'aminoacides dans laquelle chaque dégénérescence est remplacée par l'un des nucléotides de la dégénérescence, notamment T pour une dégénérescence C/T et G pour une dégénérescence G/A, et au moins une partie des groupes adénine est remplacée par des groupes adénine modifiés tels que définis plus haut.

Ces sondes constituées d'une séquence éduite d'une séquence particulière d'aminoacides et dans laquelle chaque dégénérescence est remplacée par l'un des nucléotides de la dégénérescence, notamment *T* pour une dégénérescence C/T et *G* pour une dégénérescence G/A sont décrites dans la demande de brevet FR 84 13095 dont question ci-dessus.

De préférence pour obtenir une meilleure sensibilité de détection, tous les groupes adénine du fragment d'oligonucléotide sont remplacés par des groupes adénine modifiés tels que définis plus haut.

Pour la plupart des applications, la sonde selon l'invention comporte vingt nucléotides mais un fragment plus long peut être utilisé pour des applications particulières.

La préparation de ces sondes comporte, comme étape principale, la condensation du groupe —OH en position 5' du motif sucre d'un nucléoside (1), lié par liaison covalente à un support solide, plus particulièrement un support polymère tel que du gel de silice, ou de billes de verre ou de polyacrylamide, avec (2) un dérivé de phosporamidite d'adénosine, selon le schéma suivant:

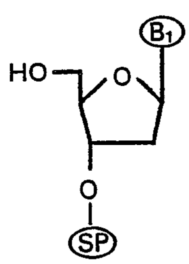

1

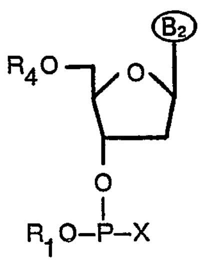

2

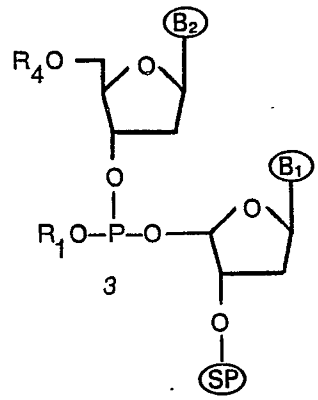

3

cette réaction est avantageusement catalysée par du 1H-tétrazole dans de l'acétonitrile.

Après oxydation et élimination du groupe protecteur en position 5' du motif sucre, on repète l'opération de condensation jusqu'à l'obtention de l'oligonucléotide de longueur désirée et on élimine éventuellement le support solide.

On notera que la séquence d'oligonucléotides de ces sondes peut renfermer conformément à la demande FR 84 13 096 du 22.08.1984, aux noms des demandeurs, en plus des dérivés ci-dessus et des bases utilisées habituellement dans la synthèse d'oligonucléotides, d'autres bases dont la présence pourrait être jugée nécessaire, notamment des bases modifiées X et/ou Y. On rappelle que ces bases modifiées donnent, au déblocage un mélange, notamment équimoléculaire selon les conditions de la réaction, d'uracyle et de cytosine ou de thymine et de méthyl-5-cytosine, d'une part, et de guanine et d'amino-2 adénosine d'autre part, selon le schéma:

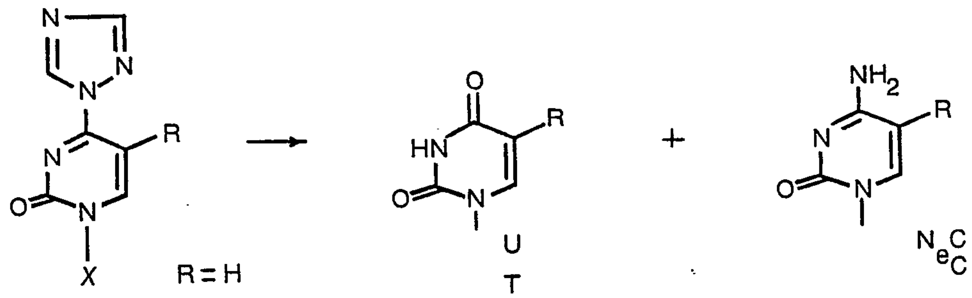

X R = H

R = $CH_3$

U

T

N C eC

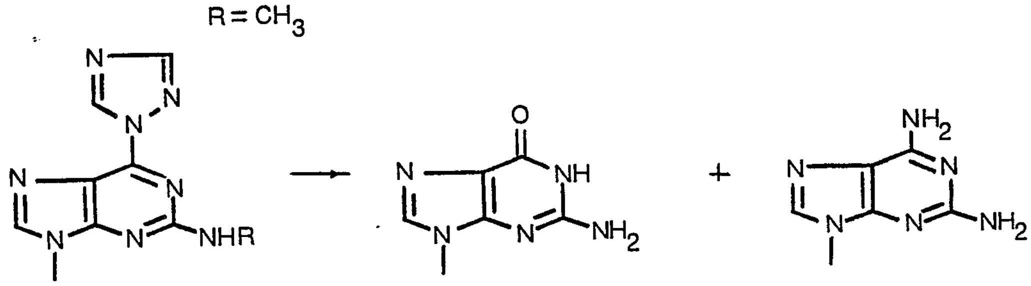

Y G A

Pour leurs applications dans le domaine des analyses et extractions biologiques, les sondes sont marquées, en vue de leur repérage après hybridation, selon les techniques classiques.

Ces sondes sont facilement accessibles par divers réactifs, notamment, par des anticorps spécifiques des bases, en particulier, des anticorps monoclonaux.

Les sondes, élaborées à partir des dérivés de l'invention, peuvent être avantageusement utilisées dans des techniques d'analyse ou d'extraction, notamment d'ARN messagers ou d'ADN complémentaires, faisant appel à des mises en évidence comportant la formation de complexes immuns, notamment, des réactions immuno-enzymatiques.

A l'aide des dérivés de phosphoramidites de l'invention, on a préparé les fragments de nucléotides appropriés pour isoler, comme décrit dans la demande de brevet FR No 84 13095, l'ADN-c de l'antithrombine III.

L'étude des sondes réalisées à l'aide de phosporamidites montre qu'elles fonctionnent comme les sondes préparées à partir des phosphotriesters de la demande de brevet FR ci-dessus.

L'invention fournit donc les moyens de mettre à profit, grâce à la réactivité élevée des dérivés de phosphoramidites élaborés, les avantages des adénines modifiées utilisées dans les sondes déjà mises au point par les demandeurs, en prticulier une meilleure stabilité des hybrides formés, une sensibilité plus élevée pour la détection après hybridation des séquences de nucléotides à analyser et la possibilité d'être utilisées avec des méthodes d'identification mettant en jeu des réactions immunologiques (immuno-enzymatiques) à la place des détections radioactives utilisées jusqu'à présent.

Dans les exemples qui suivent, on rapporte d'autres caractéristiques et avantages de l'invention.

Exemple 1

diméthoxytrityl 5′ $N_2N_6$ diisobutyrylamino-2-adénosine diisopropyl amino méthoxy phosphine-3′ de formule:

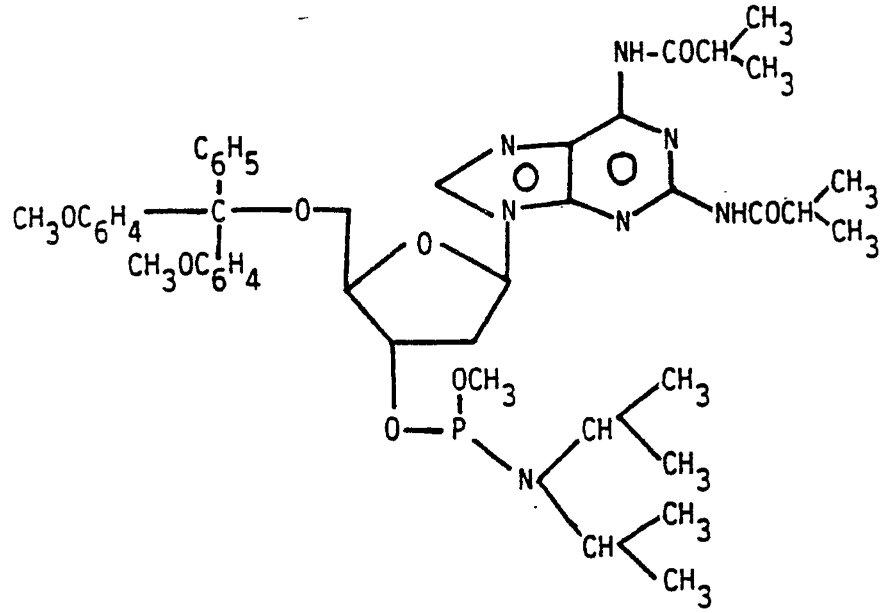

Dans un ballon de 5 ml, séché à l'étuve, on met 200 mg (0,282 mmole) de diméthoxytrityl-5′ désoxy-2′ amino-2 $N_2N_6$ diisobutyryl adénosine dissout dans 0,912 ml de dichlorométhane distillé sur $Na_2CO_3$ et 0,384 ml de diisopropylèthylamine distillée.

On ferme avec un septum après avoir chassé l'air avec de l'hélium. On adjoute alors rapidement à la seringue 180,1 mg (0,912 mmole) ce qui correspond à un excès de 3,2 de diisopropylamino chlorométhoxy phosphine. On laisse agiter 10 mn à température ambiante et on contrôle par CCM ($CH_2Cl_2$ 7,5% MeOH v:v) qu'il n'y a plus de produit de départ.

On dilue alors le mélange réactionnel dans du dichlorométhane préalablement lavé avec $NaHCO_3$ saturé. On lave très rapidement avec $NaHCO_3$ saturé, on sèche sur sulfate de sodium, on filtre et évapore à sec.

On reprend par environ 3 ml de toluène distillié et précipite par environ 60 ml d'ether de pétrole distillé refroidi à −70°C (alcool-carboglace).

La précipité restant en suspension, on centrifuge 5 mn à 5 000 t/mn. On décante le surnageant et sèche bien le précipité à la pompe à vide.

Le produit est ensuite chromatographié rapidement sur une colonne de silice 9385 Merck 2,5 g faite avec un mélange dichlorométhane/2% triéthylamine distillée pour désactiver un peu.

Elution au dichlorométhane pur. Le produit sort rapidement. Après évaporation à sec, on précipite le produit par l'éther de pétrole. Après évaporation du surnageant à sec, on obtient une poudre blanche m: 230 mg. Rdt: 93%

Rf: ($CH_2Cl_2$ 7,5% MeOH): 0.72; Rf: départ: 0,5 (AcOET 25% EP): 0,54; Rf départ: 0

MS (IE): 290 B+2; 303 DMT (IC, $NH_3$): 870 M+1, 218 $A^{iBu}$, 417 DMT désoxyribose, 580 DMT désoxyribose phosphoramidite

RMN ($CDCl_3$, $H_3PO_4$ référence interne): −149,6 ppm sextuplet, J 12,2 Hz.

Exemple 2

isolement de l'ADN complémentaire de l'antithrombine III

La séquence d'acides aminés prise en considération est la suivante:

$^{251}$Met Met Tyr Gln Glu Gly$^{256}$

ce qui correspond à la séquence d'ARN-m

5′ AUG AUG UA$^{C}_{U}$ CA$^{A}_{G}$ GA$^{A}_{G}$ G—3′

et à un ADN-c pouvant s'écrire:

5′ ATG ATG TA$^{T}_{C}$ CA$^{A}_{G}$ GA$^{A}_{G}$ G 3′

et qui comprend 3 ambiguités.

Afin d'étudier l'hybridation avec cet ADN-c on a préparé les sondes suivantes, à savoir:

à titre de témoin, une sonde mixte No. 1

3' TAC TAC AT$^{A}_{G}$ GT$^{C}_{T}$ CT$^{C}_{T}$ C 5'

une séquence selon l'invention comportant des motifs adénine modifiés:

3' T*AC T*AC *AT*G* GT*T* T*T* C 5'

Dans ces séquences *A représente un motif amino 2-dé-oxy-1 adénosine.

Les motifs soulignés — correspondent au site de dégénérescence chosi (dans le cas d'une dégénérescence C/T, oncchoisit *T* et dans le cas d'une dégénérescence G/A on choisit *G*). Ceux soulignés = correspondent à un mismatch.

Les synthèses de ces sont sont effectuées comme suit:

a) synthèse de la sonde mixte, témoin No 1. La sonde mixte a été élaborée à partir de 19 mg de résine T (résine portant le nucléotide T et préparée selon la technique de K. Itakura et coll. décrite dans Nucleic Acids Research (1980), *8*; 5473) avec les trimères suivants préparés selon la technique de K. Itakura et coll. indiquée plus haut.

```
CTT     CTT     GAT
 +       +       +      ACA     TCA     T
CCT     CCT     GGT
                                              Support solide
         ↑        ↑       ↑       ↑
Rendements:     86 %     83 %    79 %    50 %
```

Après le dernier couplage la résine est traitée par 500 µl d'un mélange molaire de pyridine aldoxime (PAO) dans la tétraméthyl guanidine (TMG) additionné de 500 µl de TMG pendant une nuit. Après évaporation à sec et chauffage avec l'ammoniaque concentrée à 50°C pendant 3 heures, le milieu réactionnel est purifié par chromatographie sur une colonne de Sephadex G—10 (dénomination commerciale de la société Pharmacia pour une colonne de polysaccharide réticulé) suivie d'une chromatographie liquide haute performance (HPLC) sur colonne en phase inverse. Le mélange d'obligonucléotides est ensuite détritylé avec l'acide acétique à 80% pendant 5 mn évaporé à sec (9 D.O.) (1 D.O. = une unité de densite optique, correspondant à 20—40 µg de séquence) et purifié par électrophorèse préparative sur gel d'acrylamide.

b) synthèse de la séquence N° 2 selon l'invention (avec A*).

La séquence contenant A* est synthétisée à partir de 30 mg de résine *T*

```
CTT   CTT   GGT   A*   C   A*   TC   A*   T
```

$^{32}P$ comme suite:

On ajoute 50 µCi de [γ-$^{32}$P]ATP sur 100 pmoles de la sonde dans un volume de 4 µl de mix et 0.5 µl de polynucléotide kinase [activité 5—20 $10^3$ unités/ml, Boehringer Mannheim]. On laisse une demi-heure à 37°C [T. Maniatis et coll. Molecular Cloning. Cold Soring Harbor Lab. (1982)]. La réaction est stoppée par addition de 1,3 µl de tampon au bleu de bromophénol 100 mM de EDTA. 50% de glycérol. Le produit marqué est purifié sur gel (0.4 × 30 × 40 cm) contenant 19 g de bisacrylamide. 19 g d'acrylamide et 10 ml de tris borate 1 M pour un volume total de 100 ml.

L'électrophorèse sous programmation: 2000 V, 43 W, 40 mA, est arrêtée quant le bleu de bromophénol atteint le tiers de la longueur de la plaque. La bande radioactive est découpée et extraite pendant une nuit dans 2 ml d'eau.

L'hybridation est effectuée comme suit:

2 colonies contenant un cADN complet de l'$AT_{III}$ et 2 colonies contenant un cADN non relaté à '$AT_{III}$ on été repiquées en 3 exemplaires sur boîtes de milieu complet avec 50 µg/ml d'ampicilline. Les colonies ont été transférées sur 3 filtres de type Whatman 4541, amplifiées 20 heures sur boîtes contenant 250 µg/ml de chloramphénicol et préparées pour l'hybridation, selon la technique décrite par J.P. Gergen et coll. dans Nucleic Acids Research, (1979), *7*, 2115. Les filtres ont été préhybridés 2 heures à 42°C en 6 NET (1 NET = 0.15 M NaCl, 0.015 M tris HCl pH = 7.5, 0.001 M EDTA), 0.5% Monidet P40 (dénomination commerciale pour un détergent), 100 µg/ml de tRNA de levure et 100 µg/ml d'ADN soniqué de sperme de saumon [technique

de T. Maniatris et coll. dans l'article cité plus haut]. L'hybridation a été effectuée à 42°C pendant 20 heures dans le même solution en présence de $10^6$ cpm d'oligonucléotide marqué en 5' au [$\gamma$-$^{32}$ P]ATP, chaque filtre ayant été hybridé avec l'une des 2 sondes. Les filtres ont été lavés 4 fois 15 minutes à 40°C en 6 SSC (1 SSC = 0.15 M NaCl, 0.015 M citrate de sodium, pH = 7.2), 0.1% de dodécyl sulfate de sodium (SDS). Les filtres ont été soumis à une autoradiographie.

On vérifie que l'hybridation est visible sur la sonde mixte *1* (1 séquence sur 8 est complémentaire du cADN) et sur la séquence spécifique *2* comportant 3 A* (1 "mismatch" et 9/16 paires de type GC).

**Revendications**

1. Nucléosides de dérivés de l'adénosine comportant des groupements adénine capables de former, après déblocage des fonctions réactives, trois liaisons hydrogène avec les groupes réactifs des bases pyrimidiques (thymine, uracile), caractérisés en ce que le motif sucre est substitué en position 3' ou en position 5' par un groupe phosphite ou phosphoramidite de formule I:

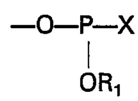

dans laquelle:

$R_1$ représente un radical alcoyle inférieur, en particulier un radical méthyle, ou $-(CH_2)_n-CN$, *n* étant un nombre de 1 à 4,

*X* représente un atome d'halogène ou un groupe amine primaire, secondaire ou tertiaire.

2. Nucléosides selon la revendication 1, caractérisés en ce que *X* représente du chlore ou du brome, ou un groupe amine tertiaire, en particulier choisi parmi les groupes diméthylamino, diéthylamino, dipropylamino, diisopropylamino, morpholino, pyrrolidino, et 2,2,6,6-tétraméthylpipéridino, ou encore un groupe amine secondaire résultant de l'élimination de l'atome d'hydrogène du cycle azoté d'un hétérocycle azoté contenant des liaisons insaturées dans le cycle tels que le tétrazole, l'imidazole, des imidazoles substitués du type du nitroimidazole, l'indole, le pyrazole, le benzimidazole, l'isoindole, le pyrrole, le triazole, le dioxazole et analogues.

3. Nucléosides selon la revendication 2, caractérisés en ce qu'ils comprennent, sur le sommet C—2 du cycle pyrimidine des groupements adénine, a) un groupe amino, b) un groupe hydroxyle —OH, ou c) un groupe sulfhydrile —SH, ces groupes étant bloqués par remplacement d'un atome d'hydrogène, ou des deux atomes d'hydrogène dans le cas du groupe amino, par un groupe protecteur tel qu'itilisé habituellement pour ces types de fonction.

4. Nucléosides selon la revendication 3, caractérisés en ce qu'ils répondent à la formule II:

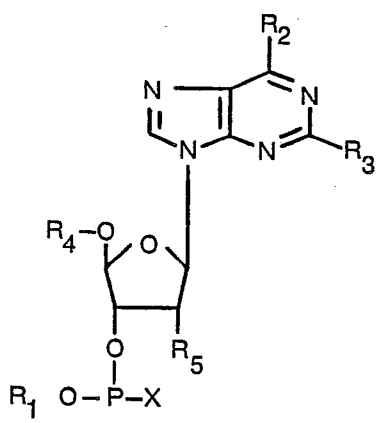

(II)

dans laquelle:

*$R_1$* et *X* sont tels que définis ci-dessus, *X* présentant avantageusement les significations préférées des différents groupes considérés,

*$R_2$* représente un groupe $-NH(A_1)$ ou $-N(A_1, A_2)$,

*$R_3$* présente les significations de *$R_2$* ou représente un groupe $-O(A_1)$ ou $-S(A_1)$, *$A_1$* et *$A_2$* identiques ou différents l'un de l'autre étant choisis parmi les groupes acyle, notamment acétyle et benzoyle, butyryle, isobutyryle et autre groupe analogue pouvant être éliminé sans altération des motifs nucléosidiques ou de la chaîne nucléotidique formée à partir de ces motifs,

*$R_4$* représente un groupe de blocage de la fonction —OH en position 5', en particulier, un groupe choisi parmi les radicaux trityle, méthoxytrityle, diméthoxytrityle, dialcoxyphosphite, pivalylisobutyloxycarbonyle, t-butyldiméthylsilyle, et

*$R_5$* représente un atome d'hydrogène ou un groupe $-OR_6$ avec *$R_6$* présentant les significations de $A_1$, $A_2$ ou $R_4$.

5. Procédé de préparation des nucléosides selon l'une quelconque des revendications 1 à 4, caractérisé en ce qu'on fait réagir à l'abri de l'air, a) un dérivé d'adénosine de formule (III) substitué par les groupements réactifs souhaités, ces groupements étant protégés et comportant un groupe —OH libre en position 3' du motif sucre, avec b) un phosphite de formule (IV), selon le schéma:

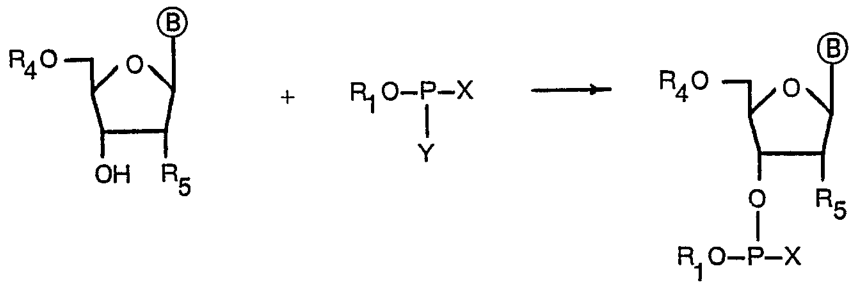

(III) (IV) (V)

dans laquelle X et $R_1$ sont tels que définis ci-dessus, Y représente un groupe réactif capable de réagir avec le groupe —OH libre du motif sucre, établissant une liaison entre O et P, et en formant un composé YH facilement éliminable et B représente une adénine modifiée, la synthèse de nucléosides renfermant un groupe phosphoramidite en position 5' étant réalisée en utilisant un nucléoside de formule III dont le groupe —OH en 5' est libre et le groupe —OH en 3' est bloqué, notamment par un substituant du type de $R_4$.

6. Procédé selon la revendication 5, caractérisé en ce que Y représente un atome d'halogène plus spécialement un atome de chlore et X représente un groupement -N,N-diisopropyle: $—N[CH(CH_3)_2]$, -N-morpholino:

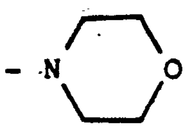,-N-pyrrolidino : 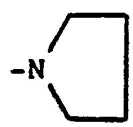 ou 2,2,6,6-tétraméthyl-N-pipéridino : 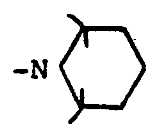

et $R_1$ représente un radical alcoyle, plus spécialement un radical méthyle ou $—(CH_2)_n—CN$, *n* étant un entier de 1 à 4, de préférence égal à 2.

7. Procédé selon la revendication 4 ou 6, caractérisé en ce que pour préparer les dérivés dans lesquels la base B comporte un groupe $—NH_2$ sur le sommet C—2, on utilise la O-diméthoxytrityl-5' N,N'-diisobutyryl amino-2 désoxy-2' adénosine pour préparer les dérivés dans lesquels la base B est modifiée sur le sommet C—2 de la pyrimidine par un groupe —OH, on utilise la 6-diméthoxy-trityl-5' N-isobutyryl hydroxy-2 désoxy-2' adénosine et que, pour préparer les dérivés dans lesquels la base B est modifiée sur le sommet C—2 par un groupe —SH, on utilise la O-diméthoxytrityl-5' N-isobutyryl mercapto-2 désoxy-2' adénosine.

8. Procédé selon l'une quelconque des revendications 5 à 7, caractérisé en ce qu'on utilise comme phosphite de formule IV un phosphochloridite choisi parmi:

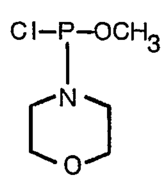

et

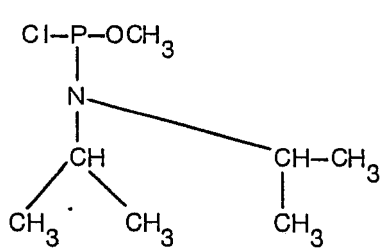

9. Application des dérivés selon l'une quelconque des revendications 1 à 4, pour l'élaboration de sondes dans le domaine des analyses et extractions biologiques par synthèse manuelle ou automatisée.

**Patentansprüche**

1. Nucleoside von Adenosinderivaten mit Adeninanteilen, die in der Lage sind, nach Entfernung der Abschirmung der reaktiven Funktionen drei Wasserstoffbindungen mit den reaktiven Gruppen der Pyrimidinbasen (Thymin, Uracil) zu bilden, dadurch gekennzeichnet, daß die Zuckerkomponente in der 3'- oder 5'-Position durch eine Phosphit- oder Phosphoramiditgruppe der Formel I

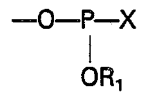

worin

$R_1$ einen niedrigen Alkylrest, insbesodnere einen Methylrest, oder

$(CH_2)_n$—CN, worin n eine ganze Zahl von 1 bis 4 ist, bedeutet,

*X* ein Halogenatom oder eine primäre, sekundäre oder tertiäre Aminogruppe bedeutet, ersetzt ist.

2. Nucleoside nach Ansprüch 1, dadurch gekennzeichnet, daß *X* ein Chlor- oder Bromatom oder eine tertiäre Aminogruppe, die insbesondere unter den Dimethylamino-, Diethylamino-, Dipropylamino-, Diisopropylamino- Morpholino-, Pyrrolidino- und 2,2,6,6-Tetramethylpiperidinogruppen ausgewählt ist, oder eine sekundäre Aminogruppe, die durch die Entfernung eines Wasserstoffatoms aus einem Stickstoffring eines Stickstoffheterozyklus mit ungesättigten Bindungen, wie Tetrazol, Imidazol, substituierte Imidazole des Nitroimidazoltyps, Indol, Pyrazol, Benzimidazol, Isoindol, Pyrrol, Triazol, Dioxazol und Analoge entsteht, bedeutet.

3. Nucleoside nach Anspruch 2, dadurch gekennzeichnet, daß sie in der C—2-Position des Pyrimidinrings der Adeninanteile a) eine Aminogruppe, b) eine Hydroxylgruppe —OH oder c) eine Sulfhydrylgruppe —SH aufweisen, wobei diese Gruppe durch die Ersetzung des Wasserstoffatoms oder zwei Wasserstoffatome im Fall der Aminogruppe durch eine Schutzgruppe des normalerweise zum Schutz dieser Funktionen verwendeten Typs geschützt sind..

4. Nucleoside nach Anspruch 3, dadurch gekennzeichnet, daß sie der Formel II

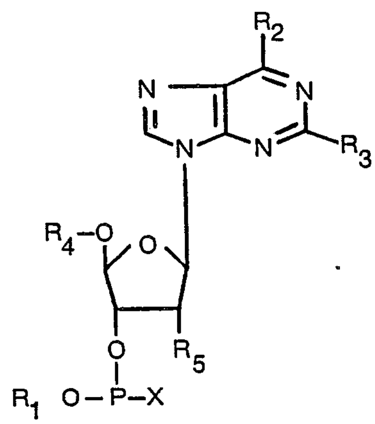

(II)

worin

$R_1$ und X die obige Bedeutung haben, wobei X vorteilhafterweise die bevorzugten Bedeutungen der Verschiedenen genannten Gruppen aufweist,

$R_2$ eine —$NH(A_1)$ oder —$N(A_1, A_2)$-Gruppe bedeutet,

$R_3$ die Bedeutungen von $R_2$ hat oder eine —$O(A_1)$- oder —$S(A_1)$-Gruppe bedeutet, worin $A_1$ und $A_2$, gleich oder verschieden, unter Acylgruppen, insbesondere Acetyl- und Benzoyl-, Butyryl-, Isobutyryl- und anderen ähnlichen Gruppen, die ohne Modifizierung der Nucleosidkomponenten oder der aus diesen Komponenten gebildeten Nucleotidkette entfernt werden können, ausgewählt sind,

$R_4$ eine Schutzgruppe für die —OH-Funktion in der 5'-Position, insbesondere eine Gruppe, die unter den Trityl-, Methoxytrityl-, Dimethoxytrityl-, Dialkoxylphosphit-, Pivalylisobutyl-oxycarbonyl-, t-Butyldimethylsilylresten ausgewählt ist, bedeutet und

$R_5$ ein Wasserstoffatom oder eine —$OR_6$-Gruppe ist, worin $R_6$ die Bedeutungen von $A_1$, $A_2$ oder $R_4$ hat, entsprechen.

5. Verfahren zur Herstellung der Nucleoside nach einem der Ansprüche 1 bis 4, dadurch gekennzeichnet, daß in der Abwesenheit von Luft a) ein Adenosinderivat der Formel (III), das durch die gewünschten reaktiven Gruppen in einer geschützten Form substituiert ist und eine freie —OH-Gruppe in 3'-Position der Zuckerkomponente hat, mit b) einem Phosphit der Formel (IV) nach dem Schema:

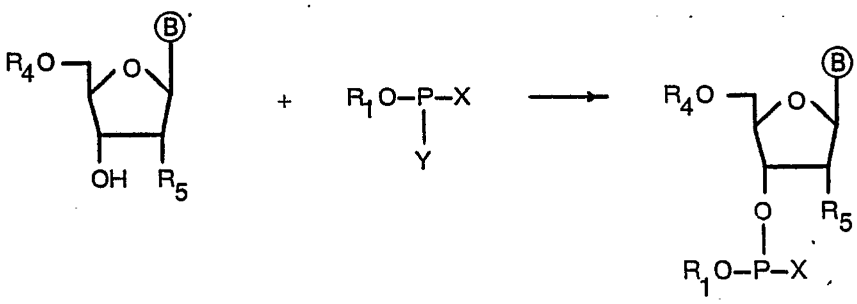

(III) (IV) (V)

worin X und $R_1$ die obige Bedeutung haben, Y eine reaktive Gruppe ist, die mit der freien —OH-Gruppe der Zuckerkomponente reagieren kann, wobei eine Bindung zwischen O und P hergestellt wird und eine leicht entfernbare YH-Verbindung entsteht, und B ein modifiziertes Adenin bedeutet, wobei die Synthese von in der 5'-Stellung ein Phosphoramidit enthaltenen Nucleosiden durch Verwendung eines Nucleosids der Formel III erreicht wird, dessen —OH-Gruppe in 5' frei ist und die —OH-Gruppe in 3' geschützt ist, insbesondere durch einen Substituenten des Typs $R_4$, zur Reaktion gebracht wird.

6. Verfahren nach Anspruch 5, dadurch gekennzeichnet, daß Y ein Halogen, insbesondere ein Chloratom, bedeutet und X eine Gruppe —N,N-Diisopropyl: $—N[CH(CH_3)_2]$, —N-Morpholino:

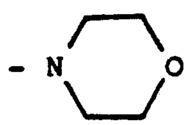,-N-pyrrolidino : 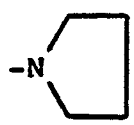 oder 2,2,6,6-tétraméthyl-N-pipéridino : 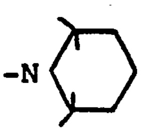

bedeutet und $R_1$ ein Alkylrest, insbesondere ein Methylrest oder $—(CH_2)_n—CN$, worin *n* eine ganze Zahl von 1 bis 4, und vorzugsweise 2, ist, ist.

7. Verfahren nach Anspruch 4 oder 6, dadurch gekennzeichnet, daß für die Herstellung der Derivate, in denen die Base B ein $—NH_2$ in der C—2-Position enthält, O-Dimethoxytrityl-5'-N,N-disobutyrylamino-2 desoxy-2'-adenosin verwendet wird, für die Herstellung der Derivate, in denen die Base B in der C—2-Position des Pyrimidins durch eine —OH-Gruppe modifiziert ist, 6-Dimethoxytrityl-5'-N-isobutyryl-hydroxy-2-desoxy-2'-adenosin verwendet wird und für die Herstellung der Derivate, in denen die Base B in der C—2-Position durch eine —SH-Gruppe modifiziert ist, O-Dimethoxytrityl-5'-N-isobutyryl-mercapto-2-desoxy-2'-adenosin verwendet wird.

8. Verfahren nach einem der Ansprüche 5 bis 7, dadurch gekennzeichnet, daß as Phosphit der Forme IV ein Phosphochloridit unter

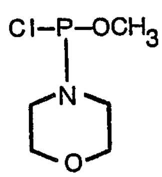 und 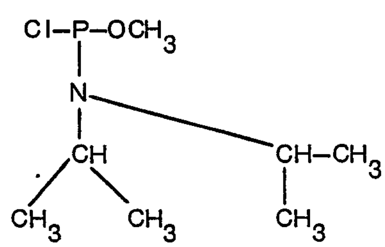

ausgewählt ist.

9. Anwendung der Derivate nach einem der Ansprüche 1 bis 4 als Sonden auf dem Gebiet der biologischen Analysen und Extraktion durch manuelle oder automatisierte Synthese.

**Claims**

1. Nucleosides of adenosine derivatives comprising adenine moieties capable of forming, after deprotection of the reactive functions, three hydrogen bonds with the reactive groups of the pyrimidine bases (thymine, uracil), characterised in that the sugar component is substituted in position 3' or 5' by a phosphite or phosphoramidite group of the formula I:

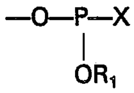

in which:

$R_1$ represents a lower alkyl radical, in particular a methyl radical or $(CH_2)_n$—CN, *n* being an integer of 1 to 4,

*X* represents a halogen atom or a primary, secondary or tertiary amino group.

2. Nucleosides according to claim 1, characterised in that *X* represents a chlorine or bromine atom, or a tertiary amino group, chosen in particular from among the groups dimethylamino, diethylamino, dipropylamino, diisopropylamino, morpholino, pyrrolidino, and 2,2,6,6-tetramethylpiperidino, or also a secondary amino group resulting from the elimination of a hydrogen atom from a nitrogen ring of a nitrogen heterocycle containing unsaturated linkages, such as tetrazole, imidazole, substituted imidazoles of the nitroimidazole type, indole, pyrazole, benzimidazole, isoindole, pyrrole, triazole, dioxazole and analogues.

3. Nucleosides according to claim 2, characterised in that they comprise at the position C—2 of the pyrimidine ring of the adenine moieties, a) an amino group, b) a hydroxyl group —OH, or c) a sulfhydryl group —SH, these groups being protected by the replacement of the hydrogen atom, or two hydrogen atoms in the case of the amino group, by a protecting group of the type normally used to protect these functions.

4. Nucleosides according to claim 3, characterised in that they correspond to the formula II:

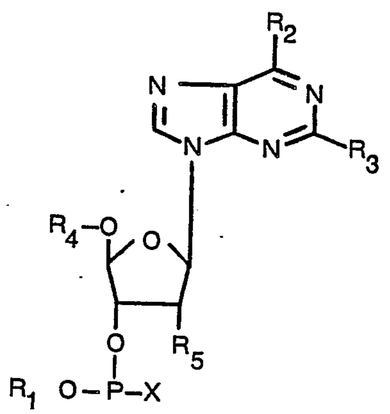

(II)

in which:

$R_1$ and X are as defined above, X advantageously representing the preferred meanings of the different groups considered,

$R_2$ represents a group $—NH(A_1)$ or $—N(A_1, A_2)$,

$R_3$ has the meanings of $R_2$ or represents a group $—O(A_1)$ or $—S(A_1)$, $A_1$ and $A_2$, identical or different, being chosen from among acyl groups, in particular, acetyl and benzoyl, butyryl, isobutyryl and other similar groups capable of being removed without modification of the nucleoside components or of the nucleotide chain formed from these components,

$R_4$ represents a protecting group for the —OH function in position 5′, in particular, a group chosen from among the radicals trityl, methoxytrityl, dimethoxytrityl, dialkoxylphosphite, pivalylisobutyloxycarbonyl, t-butyldimethylsilyl and

$R_5$ represents a hydrogen atom or a group $—OR_6$ with $R_6$ having the meanings of $A_1$, $A_2$, or $R_4$.

5. Process of preparation of the nucleosides according to any one of the claims 1 to 4, characterised in that, in the absence of air, are made to react a) an adenosine derivative of formula (III), substituted by the desired reactive groups in a protected form and bearing a free —OH group in position 3′ of the sugar component, with b) a phosphite of formula (IV), according to the scheme:

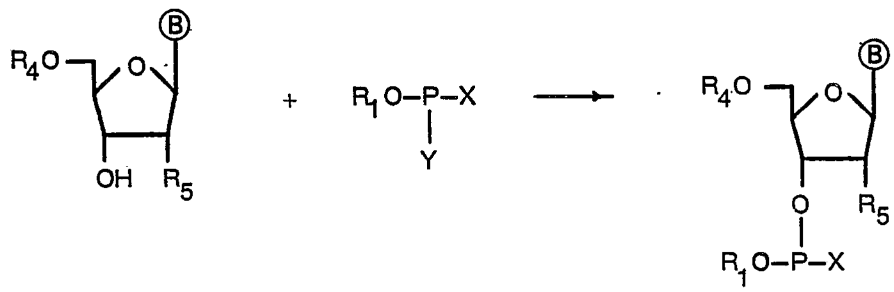

(III) (IV) (V)

in which X and $R_1$ are as defined above, Y represents a reactive grouping capable of reacting with the free —OH group of the sugar component, leading to the establishment of a bond between O and P and giving rise to a readily removable compound YH, and B represents a modified adenine, the synthesis of nucleosides containing a phosphoramidite in position 5′ being achieved by using a nucleoside of formula III, the —OH group of which at 5′ is free and the —OH group at 3′ is protected, in particular by a substituent of type $R_4$.

6. Process according to claim 5, characterised in that Y represents a halogen, more especially a chlorine atom and X represents a group —N,N-diisopropyl: $—N[CH(CH_3)_2]$, —N-morpholino:

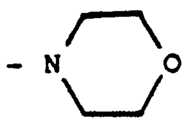 ,-N-pyrrolidino : 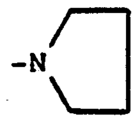 or 2,2,6,6-tetramethyl-N-piperidino : 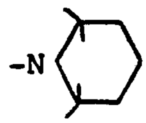

and $R_1$ represents an alkyl radical, more particularly a methyl radical or $—(CH_2)_n—CN$, *n* being an integer from 1 to 4, and, preferably, 2.

7. Process according to claim 4 or 6, characterised in that the preparation of the derivatives in which the base B contains an $—NH_2$ at position C—2, O-dimethoxytrityl-5′ N,N-diisobutyryl amino-2 desoxy-2′ adenosine is used, for the preparation of the derivatives in which the base B is modified at the position C—2 of the pyrimidine by a —OH group, 6-dimethoxytrityl-5′ N-isobutyryl hydroxy-2 desoxy-2′ adenosine is used and for the preparation of the derivatives in which the base B is modified at position C—2 by an —SH group, O-dimethoxytrityl-5′ N-isobutyryl mercapto-2 desoxy-2′ adenosine is used.

8. Process according to any one of the claims 5 to 7, characterised in that, as the phosphite of formula IV, a phosphochloridite is chosen among:

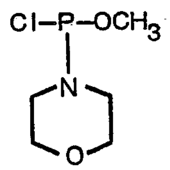 and 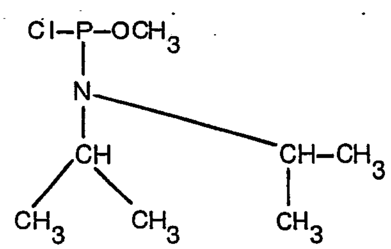

9. Application of the derivatives according to any one of the claims 1 to 4 as probes in the field of biological analyses and extraction by manual or automatized synthesis.